# EUROPEAN PATENT APPLICATION

(11) **EP 1 911 439 A2**
(43) Date of publication of application: **16.04.2008**
(21) Application number: 07113629.5
(22) Date of filing: 01.08.2007
(51) Int. Cl.: A61K 8/73, A61Q 19/00

(54) **Solid cosmetic and therapeutic compositions applicable to the human skin and gellable on contact with water**

(30) Priority: 09.10.2006 IT MI20061933
(71) Applicant: BIOFARMITALIA S.p.A., 20144 Milano (IT)
(72) Inventor: Lombardo, Paola, 24060 Costa Mezzate (BG) (IT)
(74) Representative: Frignoli, Luigi

(57) **Abstract**

Solid compositions applicable to the human skin and gellable on contact with water. These compositions are prepared in the form of a flexible solid film and can be used for cosmetic and/or therapeutic purposes. The compositions contain hyaluronic acid, a very fine inert powder and a small quantity of water as essential components.

## Description

Traditional cosmetic and pharmaceutical compositions for dermal application or absorption consist generally of emulsions, gels, lotions, unguents etc. By their nature, these compositions require the presence in them of large water quantities, defined as AW ≤ 0.6 (where AW indicates the ratio of vapour pressure of the water present in the composition to the pressure of pure water at the same temperature): for this reason the compositions require preservatives which in certain concentrations could be poorly tolerated by the body. Moreover the marketing of these compositions requires them to be packaged in multidose containers (bottles, jars, tubes etc.) which, once opened, become an easy terrain for external contamination by bacteria and moulds: single dose packages are very costly as the packaging cost represents a particularly high proportion of an individual dose.

The main object of the present invention is to obviate the aforestated drawbacks by providing solid cosmetic and/or pharmaceutical compositions with a low quantity of free water, which can be produced in single doses for single applications and which are as valid, effective and pleasant to use as traditional preparations, a plurality of separate single doses being able to be enclosed and preserved in the same container.

The compositions of the present invention are characterised by being in the solid state gellable on contact with water and comprising between 6.0% and 40% of hyaluronic acid or its alkaline salts, between 5% and 70% of an inert powder of particle size less than 150 micron, and between 6.0% and 40% of bound water, the percentages being by weight on the weight of the solid compositions.

The term "bound water" indicates water which is not freely available because it is bound covalently to the ions of the other molecules forming part of the same composition.

Advantageously, the solid compositions of the present invention also comprise at least one further component chosen from the group consisting of emulsifying, wetting and pharmaceutical substances; said inert powder is chosen from the group consisting of mica, talc, silica, polymethyl methacrylate, lauroyl lysine and corn starch powders; and said emulsifying substances are chosen from the group consisting of partial esters of sorbitan polyethoxylate with fatty acids, and partial esters of glycerol polyethoxylate with fatty acids.

Again advantageously, the solid compositions of the invention are prepared in the form of solid films of thickness between 60 and 150 microns which are gellable, i.e. which are able to transform into a gel if imbibed with a water quantity even less than 1 ml per cm².

This gel, flowable and easily absorbable, is able to carry cosmetic and/or pharmaceutical active principles.

Said solid films, of good dimensional stability, can be punched or cut into the most suitable shapes for their cosmetic and/or pharmaceutical use.

The aforesaid solid compositions can be prepared by a method according to which hyaluronic acid or its sodium salts are dispersed in a mixer containing water preheated to between 50°C and 70°C, which is then agitated until a clear gel free from lumps is obtained, an inert powder and other possible components to be present in the solid compositions then being added slowly into the same mixer by trickling, and slow agitation continued until the powder is completely dispersed, slow cooling then being commenced under agitation to a temperature between 20°C and 30°C until a homogeneous mass is obtained which, using a doctor blade, is spread in the form of a film of between 180 and 270 micron thickness onto a siliconized surface of a support belt which is passed through a ventilated tunnel oven with successive heating stations of increasing temperatures to a maximum of 120°C, the last station being at a lower temperature between 60°C and 90°C, at the oven exit a solid film of between 60 and 150 micron thickness being detached from the support belt and cooled to ambient temperature, then punched into the required shape and size and divided into unit portions of the desired solid compositions.

To optimise the aforestated process, the heated tunnel oven must have at least one variable temperature drying station, better still four stations, the first of which is heated to a temperature between 60°C and 80°C, the second to a temperature between 70°C and 90°C, the third between 100°C and 120°C, and the fourth between 70°C and 90°C.

The unit portions obtained in this manner can then be inserted into the final packages from which they can be withdrawn at the moment of use.

Some non-limiting embodiments will now be described to clarify the understanding of the characteristics of the solid compositions of the invention and the method for their preparation.

### EXAMPLE 1

### Preparation of a solid film with a hydrating and filling effect on cutaneous microprotuberances

To prepare a solid composition of the present invention the following components are used:

| **COMPONENT** | **FUNCTION/DESCRIPTION** | **QUANTITY BY WEIGHT** |
|---|---|---|
| 1) Water | Solvent | 83.5 Kg |
| 2) Hyauronic acid | Filmogen / MW 1,200,000 | 1.5 Kg |
| | | |
| 3) Lauroyl lisine | Inert powder/ Particle size < 100 micron | 15 Kg |

### Operative method

1) Component 2 (hyaluronic acid) is dispersed under agitation in a mixer containing water preheated to between 50°C and 70°C; agitation is applied until a clear gel free of lumps is obtained.
2) After verifying that the hyaluronic acid has completely dispersed, component 3 is slowly trickled in, then the mixture is agitated with slow agitation for 30 min until the inert powder has completely dispersed.
3) Slow cooling is commenced under agitation to 25°C; agitation is continued until homogeneity is attained.
4) Still under agitation, using a peristaltic pump the mixture obtained is fed onto a doctor blade heated to 30°C and then filmed to 200 micron thickness on a siliconized polyester belt support. During filming, a ventilated tunnel oven is used provided with four heating stations having the following temperatures respectively: 80/85/100/80°C.
5) At the oven exit, a film having a thickness of 70 micron (following water evaporation) separates automatically from the polyester belt support.
6) The film is punched into rectangles of 3 x 4 cm using a roller punch; punching can be effected while leaving the film resting on and adhering to the support belt.
7) These rectangles are automatically inserted into a sealed container acting as the dispensing mechanism for the solid composition.

### Product specifications

| | |
|---|---|
| Weight of one unit of dimensions 3 x 4 cm | 68 mg |
| Thickness | 70 micron |
| AW (Water Activity), i.e. the ratio of vapour pressure of the water present in the composition to the pressure of pure water at the same temperature) | 0.3 |
| Bound water | 33.61% |
| Hyaluronic acid | 6.03% |
| Lauroyl lysine | 60.36% |

(the percentages are by weight on the total product weight).

To verify the applicational functionality of the solid composition obtained, an in vivo test is carried out: a rectangular film piece of the composition of dimensions 4 x 3 cm is deposited on the rear of the previously wetted forearm of six persons of female sex. This film piece is soaked with 1 ml of water then, while making rapid circular movements, complete gelling and disintegration with subsequent absorption is awaited, with a consequent hydrating effect on the skin and filling of the cutaneous microprotuberances.

The data obtained in this manner are collected in a table and compared with the previously preset parameters as indicators of product conformation (film dissolution time expressed in seconds, absence of residues on the cutis, tactile sensation).

### EXAMPLE 2

### Preparation of a solid film with a hydrating and filling effect on cutaneous microprotuberances

To prepare the solid composition the following components are used:

| **COMPONENT** | **FUNCTION/DESCRIPTION** | **QUANTITY BY WEIGHT** |
|---|---|---|
| 1) Water | Solvent | 80.2 Kg |
| 2) Hyauronic acid | Filmogen / MW 50,000 | 9.8 Kg |
| | | |
| 3) Lauroyl lisine | Inert powder/ Particle size < 100 micron | 10 Kg |

### Operative method

1) Component 2 (hyaluronic acid) is dispersed under agitation in a mixer containing water preheated to between 50°C and 70°C; agitation is applied until a clear gel free of lumps is obtained.
2) After verifying that the hyaluronic acid has completely dispersed, component 3 is slowly trickled in, then the mixture is agitated with slow agitation for 30 min until the inert powder has completely dispersed.
3) Slow cooling is commenced under agitation to 25°C and agitation is continued until homogeneity is obtained.
4) Still under agitation, using a peristaltic pump the mixture obtained is fed onto a doctor blade heated to 30°C and then filmed to 200 micron thickness on a siliconized polyester belt support. During filming, a ventilated tunnel oven is used provided with four heating stations having the following temperatures respectively: 80/85/100/80°C.
5) At the oven exit, a film having a thickness of 70 micron (following water evaporation) separates automatically from the polyester belt support.
6) The film is punched into rectangles of 3 x 4 cm using a roller punch; punching can be effected while leaving the film resting on and adhering to the support belt.
7) These rectangles are automatically inserted into a sealed container acting as the dispensing mechanism for individual film rectangles.

### Product specifications

| | |
|---|---|
| Weight of one unit of dimensions 3 x 4 cm | 68 mg |
| Thickness | 70 micron |
| AW (Water Activity) | 0.3 |
| Bound water | 28.84% |
| Hyaluronic acid | 35.22% |
| Lauroyl lysine | 35.94% |

To verify the applicational functionality of the solid composition obtained, an in vivo test is carried out: a rectangular film piece dimensions 4 x 3 cm is deposited on the rear of the previously wetted forearm of 6 persons of female sex. This film piece is soaked with 1 ml of water then, while making rapid circular movements, complete gelling and disintegration with subsequent absorption is awaited, with a consequent hydrating effect on the skin and filling of the cutaneous microprotuberances.

The data obtained in this manner are collected in a table and compared with the previously preset parameters as indicators of product conformation (film dissolution time expressed in seconds, absence of residues on the cutis, tactile sensation).

### EXAMPLE 3

### Preparation of a solid film with a hydrating and filling effect on cutaneous microprotuberances

To prepare the desired solid composition the following components are used:

| **COMPONENT** | **FUNCTION/DESCRIPTION** | **QUANTITY BY WEIGHT** |
|---|---|---|
| 1) Water | Solvent | 82.5 Kg |
| 2) Hyauronic acid | Filmogen / MW 1,000,000 | 5.0 Kg |
| 3) Corn starch | Technological coadjuvant | 2.5 Kg |
| 4) Lauroyl lisine | Inert powder/ Particle size < 100 micron | 10.0 Kg |

### Operative method

1) Component 2 (hyaluronic acid) is dispersed under agitation in a mixer containing water preheated to between 50°C and 70°C; agitation is continued until a clear gel free of lumps is obtained.
2) After verifying that the hyaluronic acid has completely dispersed, components 3 and 4 are slowly trickled in, then slow agitation is continued for 30 min until the inert powder has completely dispersed.
3) Slow cooling is commenced under agitation to 25°C and agitation is continued until homogeneity is obtained.
4) Still under agitation, using a peristaltic pump the mixture obtained is fed onto a doctor blade and then filmed to 200 micron thickness on a siliconized polyester belt support. During filming, a ventilated tunnel oven is used provided with four heating stations having the following temperatures respectively: 80/85/100/80°C.
5) At the oven exit, a film having a thickness of 70 micron (by water evaporation) separates automatically from the polyester belt support.
6) The film is punched into rectangles of 3 x 4 cm using a roller punch; punching can be effected while leaving the film resting on and adhering to the support belt.
7) These rectangles are automatically inserted into a sealed container acting as the dispensing mechanism for individual rectangular doses of the solid composition.

### Product specifications

| | |
|---|---|
| Weight of one unit of dimensions 3 x 4 cm | 68 mg |
| Thickness | 70 micron |
| AW (Water Activity) | 0.5 |
| Bound water | 32.0% |
| Hyaluronic acid | 19.5% |
| Corn starch | 9.7% |
| Lauroyl lysine | 38.8% |

To verify the applicational functionality of the solid composition obtained, an in vivo test is carried out: a rectangular film piece of dimensions 4 x 3 cm is deposited on the rear of the previously wetted forearm of 6 persons of female sex. This film piece is soaked with 1 ml of water then, while making rapid circular movements, complete gelling and disintegration with subsequent absorption is awaited, with a hydrating effect on the skin and filling of the cutaneous microprotuberances.

The data obtained in this manner are collected in a table and compared with the previously preset parameters as indicators of product conformation (film dissolution time expressed in seconds, absence of residues on the cutis, tactile sensation).

### EXAMPLE 4

### Preparation of a solid film with a hydrating and filling effect on cutaneous microprotuberances

To prepare the desired solid composition the following components are used:

| **COMPONENT** | **FUNCTION/DESCRIPTION** | **QUANTITY BY WEIGHT** |
|---|---|---|
| 1) Water | Solvent | 46 Kg |
| 2) Hyauronic acid | Filmogen / MW 1,500,000 | 8.0 Kg |
| 3) Lauroyl lisine | Inert powder/ Particle size < 100 micron | 26 Kg |
| 4) Polysorbate-80 | Emulsifier | 8 Kg |
| 5) Glycerin | Wetting agent | 6 Kg |
| 6) Corn starch | Technological coadjuvant | 6 Kg |

### Operative method

1) Component 2 (hyaluronic acid) is dispersed under agitation in a mixer containing water preheated to between 50°C and 70°C; agitation is continued until a clear gel free of lumps is obtained.
2) After verifying that the hyaluronic acid has completely dispersed, component 3 and then, in sequence, components 4, 5 and 6 are slowly trickled in; slow agitation is continued until the inert powder has completely dispersed.
3) Slow cooling is commenced under agitation to 25°C; agitation is continued until homogeneity is obtained.
4) Still under agitation, using a peristaltic pump the mixture obtained is fed onto a doctor blade and then filmed to 200 micron thickness on a siliconized polyester belt support. During filming, a ventilated tunnel oven is used provided with four heating stations having the following temperatures respectively: 60/75/110/70°C.
5) At the oven exit, a film having a thickness of 70 micron (by water evaporation) separates automatically from the polyester belt support.
6) The solid film is punched into rectangles of 3 x 4 cm using a roller punch; punching can be effected while leaving the film resting on and adhering to the support belt.
7) These rectangles are automatically inserted into a sealed container acting as the dispensing mechanism for individual film rectangles.

### Product specifications

| | |
|---|---|
| Weight of one unit of dimensions 3 x 4 cm | 72 mg |
| Thickness | 70 micron |
| AW (Water Activity) | 0.4 |
| Bound water | 7.8% |
| Hyaluronic acid | 13.6% |
| Polysorbate-80 | 13.6% |
| Corn starch | 10.3% |
| Lauroyl lysine | 44.4% |
| Glycerin | 10.3% |

To verify the applicational functionality of the solid composition obtained, an in vivo test is carried out: a film piece is deposited on the rear of the previously wetted forearm of 6 persons of female sex. This film piece is soaked with 1 ml of water then, while making rapid circular movements, complete gelling and disintegration with subsequent absorption is awaited, (with a hydrating effect on the skin and filling of the cutaneous microprotuberances).

The data obtained in this manner are collected in a table and compared with the previously preset parameters as indicators of product conformation (film dissolution time expressed in seconds, absence of residues on the cutis, tactile sensation).

### EXAMPLE 5

### Preparation of a solid film with a hydrating and lifting effect

To prepare the desired solid composition the following components are used:

| **COMPONENT** | **FUNCTION/DESCRIPTION** | **QUANTITY BY WEIGHT** |
|---|---|---|
| 1) Water | Solvent | 49 Kg |
| 2) Hyauronic acid | Filmogen / MW 1,500,000 | 6.0 Kg |
| 3) Polymethyl methacrylate | Inert powder/particle size < 100 micron | 21.0 Kg |
| 4) Lauroyl lisine | Inert powder/ Particle size < 100 micron | 6.0 Kg |
| 5) Polysorbate-60 | Emulsifier | 6.0 Kg |
| 6) Butylene glycol | Wetting agent | 6.0 Kg |
| 7) Corn starch | Technological coadjuvant | 6.0 Kg |
| 8) Hydroxypropyl cyclodextrin | Active ingredient | 3.0 Kg |

### Operative method

1) Component 2 (hyaluronic acid) is dispersed under agitation in a mixer containing water preheated to between 50°C and 70°C; agitation is continued until a clear gel free of lumps is obtained.
2) After verifying that the hyaluronic acid has completely dispersed, component 3 and then, in sequence, components 4, 5, 6, 7 and 8 are slowly trickled in; the treatment is continued under slow agitation for 30 min until homogeneity is achieved.
3) Slow cooling is commenced under agitation to 25°C; agitation is continued until homogeneity is achieved.
4) Still under agitation, using a peristaltic pump the mixture obtained is fed onto a doctor blade and then filmed to 200 micron thickness on a siliconized polyester belt support. During filming, a ventilated tunnel oven is used provided with four heating stations having the following temperatures respectively: 80/90/120/90°C.
5) At the oven exit, a film having a thickness of 85 micron (by water evaporation) separates automatically from the polyester belt support.
6) The solid film is punched into rectangles of 3 x 4 cm using a roller punch; punching can be effected while leaving the film resting on and adhering to the belt support belt.
7) These rectangles are automatically inserted into a sealed container acting as the dispensing mechanism for individual solid film portions.

### Product specifications

| | |
|---|---|
| Unit weight of 3 x 4 cm rectangles | 98 mg |
| Thickness | 85 micron |
| AW (Water Activity) | 0.3 |
| Bound water | 8.8% |
| Hyaluronic acid | 10.7% |
| Polymethyl methacrylate | 37.6% |
| Lauroyl lysine | 10.7% |
| Polysorbate-80 | 10.7 % |
| Butylene glycol | 10.7 % |
| Corn starch | 5.4 % |
| Hydroxypropyl cyclodextrin | 5.4 % |

To verify the applicational functionality of the solid composition obtained, an in vivo test is carried out: a film piece is deposited on the rear of the previously wetted forearm of 6 persons of female sex. This film piece is soaked with 1 ml of water then, while making rapid circular movements, complete gelling and disintegration with subsequent absorption is awaited, (with a hydrating effect on the skin and filling of the cutaneous microprotuberances).

The data obtained in this manner are collected in a table and compared with the previously preset parameters as indicators of product conformation (film dissolution time expressed in seconds, absence of residues on the cutis, tactile sensation).

### EXAMPLE 6

### Preparation of a solid film with analgesic action

To prepare the desired solid composition the following components are used:

| **COMPONENT** | **FUNCTION/DESCRIPTION** | **QUANTITY BY WEIGHT** |
|---|---|---|
| 1) Water | Solvent | 65.5 Kg |
| 2) Hyauronic acid | Filmogen / MW 400,000 | 4.0 Kg |
| 3) Sulphonic polymer, ammonium salt | Filmogen | 2.0 Kg |
| 4) Polymethyl methacrylate | Inert powder/particle size < 100 micron | 20.0 Kg |
| 5) Lauroyl lisine | Inert powder/ Particle size < 100 micron | 20.0 Kg |
| 6) Glycerin | Wetting agent | 2.5 Kg |
| 7) Butylene glycol | Wetting agent | 2.5 Kg |
| 8) Polysorbate-80 | Emulsifier | 3.0 Kg |
| 9)Lidocain hydrochloride | Active ingredient | 0.5 Kg |

### Operative method

1) Component 2 (hyaluronic acid) is dispersed under agitation in a mixer containing water preheated to between 50°C and 70°C; agitation is continued until a clear gel free of lumps is obtained.
2) After verifying that the hyaluronic acid has completely dispersed, component 3 is then slowly trickled in; mixing is continued under slow agitation for 30 min until homogeneity is achieved.
3) Components 4, 5, 6 and 7 are introduced in sequence; mixing is continued under slow agitation for 30 min until homogeneity is achieved.
4) Slow cooling is commenced under agitation, and at 25°C component 9, previously dispersed in part of the water of the composition, is introduced; agitation is continued.
5) Still under agitation, using a peristaltic pump the mixture obtained is fed onto a doctor blade and then filmed to 200 micron thickness on a siliconized polyester belt support. During filming, a ventilated tunnel oven is used provided with four heating stations having the following temperatures respectively: 80/85/100/80°C.
6) At the oven exit, a film having a thickness of 80 micron (by water evaporation) separates automatically from the polyester belt support.
7) The product is punched into rectangles of 3 x 4 cm using a roller punch; punching can be effected while leaving the film resting on and adhering to the belt support belt.
8) These rectangles are automatically inserted into a sealed container acting as the dispensing mechanism for individual rectangles.

### Product specifications

| | |
|---|---|
| Unit weight of 3 x 4 cm rectangles | 75 mg |
| Thickness | 80 micron |
| AW (Water Activity) | 0.3 |
| Bound water | 10.7 % |
| Hyaluronic acid | 6.6 % |
| Sulphonic polymer, ammonium salt | 3.2 % |
| Polymethyl methacrylate | 32.8% |
| Glycerin | 4.1 % |
| Butylene glycol | 4.1 % |
| Polysorbate-80 | 4.9 % |
| Lidocain hydrochloride | 0.8 % |
| Lauroyl lysine | 32.8% |

To verify the applicational functionality of the solid composition obtained, an in vivo test is carried out: a film piece is deposited on the rear of the previously wetted forearm of 6 persons of female sex. This film piece is soaked with 1 ml of water then, while making rapid circular movements, complete gelling and disintegration with subsequent absorption is awaited, with consequent analgesic effect).

The data obtained in this manner are collected in a table and compared with the previously preset parameters as indicators of product conformation (film dissolution time expressed in seconds, absence of residues on the cutis, tactile sensation).

## Claims

1. Cosmetic and therapeutic compositions applicable to the human skin, **characterised by** being in the solid state gellable on contact with water and comprising between 6.0% and 40% of hyaluronic acid or its alkaline salts, between 5% and 70% of an inert powder of particle size less than 150 micron, and between 6.0% and 40% of bound water, the percentages being by weight on the weight of the solid compositions.

2. . Compositions as claimed in claim 1, **characterised by** comprising at least one further component chosen from the group consisting of emulsifying, wetting and pharmaceutical substances.

3. ompositions as claimed in claim 1, **characterised in that** said inert powder is chosen from the group formed from mica, talc, silica, polymethyl methacrylate, lauroyl lysine and corn starch powders.

4. . Compositions as claimed in claim 2, **characterised in that** and said emulsifying substances are chosen from the group consisting of partial esters of sorbitan polyethoxylate with fatty acids, and partial esters of glycerol polyethoxylate with fatty acids.

5. . A method for preparing solid compositions gellable with water, according to which hyaluronic acid or its sodium salts are dispersed in a mixer containing water preheated to between 50°C and 70°C, which is then agitated until a clear gel free from lumps is obtained, an inert powder and other possible components to be present in the solid compositions then being added slowly into the mixer by trickling, and slow agitation continued until the powder is completely dispersed, slow cooling then being commenced under agitation to a temperature between 20°C and 30°C until a homogeneous mass is obtained which, using a doctor blade, is spread in the form of a film of between 180 and 270 micron thickness onto a siliconized surface of a support belt which is passed through a ventilated tunnel oven with successive heating stations of increasing temperatures to a maximum of 120°C, the last station being at a lower temperature between 60°C and 90°C, at the oven exit a solid film of between 60 and 150 micron thickness being detached from the support belt and cooled to ambient temperature, then punched into the required shape and size and divided into unit portions of the desired solid compositions.
